# EUROPEAN PATENT SPECIFICATION

(11) **EP 0 390 613 B1**
(45) Date of publication and mention of the grant of the patent: **10.08.1994**
(21) Application number: 90303523.6
(22) Date of filing: 02.04.1990
(51) Int. Cl.: A61B 17/08, A61B 17/064

(54) **Absorbable surgical fastener with bone penetrating elements**
Absorbierbare chirurgische Klammer mit knochendurchdringenden Elementen
Agrafe chirurgicale résorbable avec éléments pénétrant dans un os

(30) Priority: 31.03.1989 US 332355
(43) Date of publication of application: 03.10.1990
(73) Proprietor: United States Surgical Corporation, Norwalk, Connecticut 06856 (US)
(72) Inventor: Sander, Thomas W., Newtown, CT 06482 (US); White, Jeffrey S., Ridgefield, CT 06877 (US)
(74) Representative: Marsh, Roy David

(56) References cited:
- EP-A- 0 127 994
- EP-A- 0 273 872
- EP-A- 0 283 127
- EP-A- 0 346 033
- DE-A- 2 721 075
- US-A- 3 739 773
- US-A- 3 797 499

## Description

This invention relates to surgical fasteners for fastening body tissue and more particularly relates to an at least partially absorbable fastener for fastening bone or hard tissue.

Bone fastening or fixation devices are well known in the art. Typically, such fasteners are in the form of staples, pins, screws, and wires. For example, both Pratt et al., U.S. Patent No. 4,454,875 and Ellison et al., U.S. Patent No. 4,570,623 disclose staples for being driven into bones. Staples of this type are generally fabricated of biologically inert metal, such as stainless steel, titanium, cobalt-chromium-molybdenum alloys and the like. The staple must be relatively strong and hard so that it can be easily driven directly into bone or hard tissue.

Other metal fasteners are well known commercial products used for a wide variety of bone fixation procedures. Pins and wires are generally constructed from stainless steel and are grasped in a drill chuck and self-drilled directly into bone to treat a given traumatic or pathological condition.

The disadvantage of metal fasteners is that after they have completed their function of supporting the bone while the bone heals, they remain permanently in the body. Problems can arise after healing, for example, by corrosion of the metal, or when the pins or staples work loose from their moorings and migrate through body tissue.

Furthermore, permanent metal fixation devices shield the bone from beneficial stresses after healing. It has been shown that moderate periodic stress on bone tissue, such as the stress produced by exercise, helps to prevent decalcification of the bone. Under some conditions, the stress shielding which results from the long term use of metal bone fixation devices can lead to osteoporosis.

These disadvantages can be mitigated by the use of bioabsorbable surgical fasteners, which degrade over a period of time thereby gradually transferring more support load to the bone as it heals. Such fasteners for bone are also known in the art. For instance, Tunc, U.S Patent No. 4,539,981 teaches the use of polymers of L(-)lactide for fabricating bone fixation devices. Moreover, various types of bioabsorbable pin fasteners have been commercialized. For example, some types of pins are fabricated from poly (p-dioxanone) and are indicated for use to fix in place small bony fragments in the knee and hand, where such fragments are not in tension. As is characteristic for all such absorbable pins, holes must be previously drilled into the bone in order for the pins to be inserted. Bioabsorbable fasteners are not self-inserting, i.e. they are not capable of being driven or screwed directly into bone because the polymeric material they are made of is relatively soft. The necessity to predrill holes in the injured bone adds to the surgical procedures and lengthens the time required to complete the operation.
US-A-3739773 discloses solid prosthetic devices (which expression includes pins) made of polyglycolic acid (PGA) and states that the absorbability and stiffners of the PGA can be varied. Specifically mentioned is a bone nail of PGA, to be driven into a bone structure.
US-A-3797499 discloses pins and screws machined from cast polylactide material.
EP-A-0346033 is part of the state of the art only by virtue of Article 54(3)EPC. It discloses a bone pin comprising a cutting device which may be of metal, secured to the smaller end of a tapered portion of polymeric material.

Up to now, there has been no surgical bone fastener which retained the advantages of the above mentioned types of fasteners, without the concurrent disadvantages.

Accordingly, it is one object of the present invention to provide a surgical fastener for bone or hard tissue.

It is another object of the present invention to provide a fastener which can be implanted directly into bone or hard tissue.

It is yet another object of the present invention to provide a surgical fastener which is at least partially bioabsorbable.

These and further objects are achieved herein by providing a surgical fastener, said surgical fastener comprising a bioabsorbable fastening body portion having at least one bone penetrating metallic or ceramic tip, and further by implanting said surgical fastener into segments of bone or hard tissue, said bioabsorbable fastening body portion maintaining said segments in close adjacency for a period of time sufficient to promote healing.

The drawings of this specification show embodiments of the invention. In the drawings:
Fig. 1A illustrates an exploded perspective view of the staple type bone fastener of the present invention;
Figs. 1B, 1C and 1D illustrate, respectively, top, side, and bottom views or the staple type bone fastener of the present invention;
Fig. 1E illustrates the staple type bone fastener in conjunction with a pusher mechanism of an applying instrument;
Fig. 2 illustrates an exploded side view of a pin type fastener of the present invention;
Fig. 3 illustrates an end view of the trocar point of the present invention;
Fig. 4 shows an alternative embodiment of the present invention illustrating alternative tip and mounting fixtures; and,
Fig. 5 illustrates another embodiment of the mounting fixture.

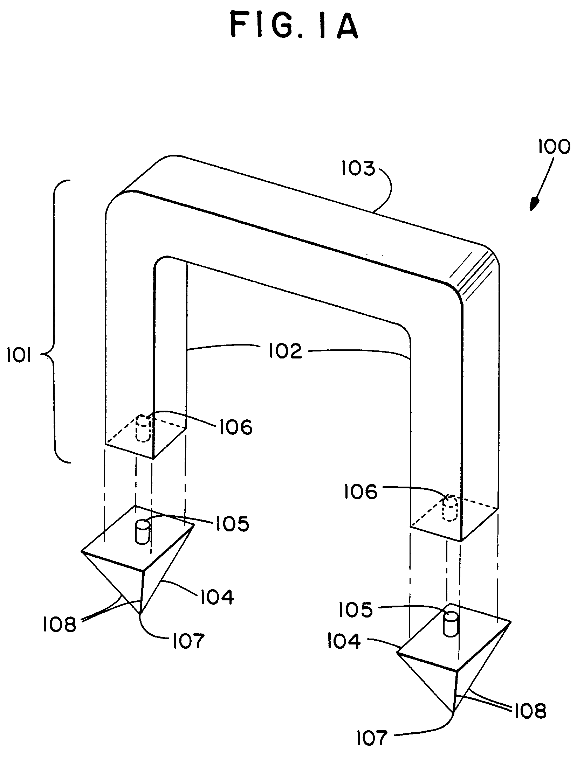

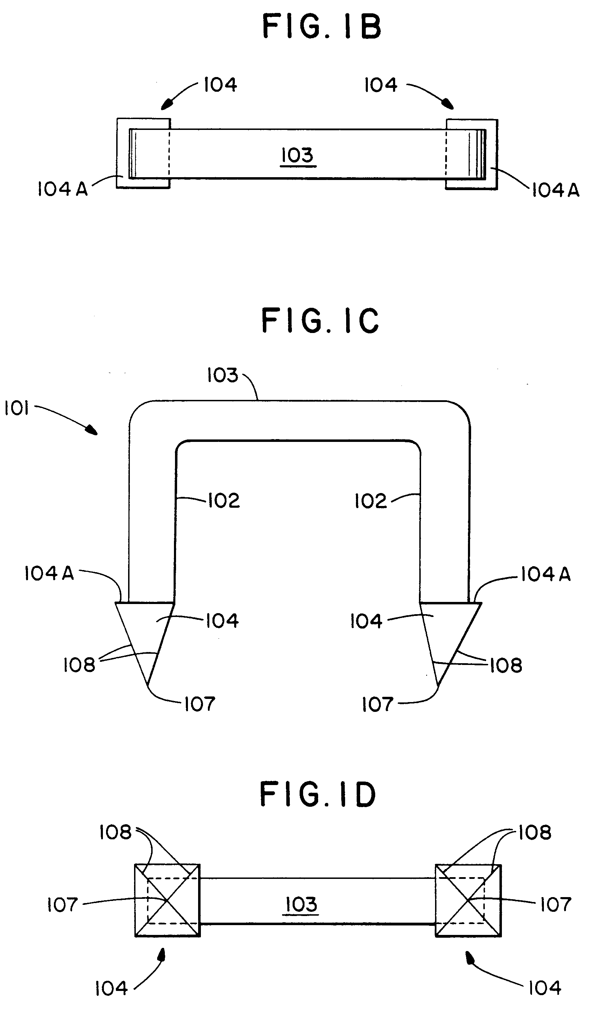

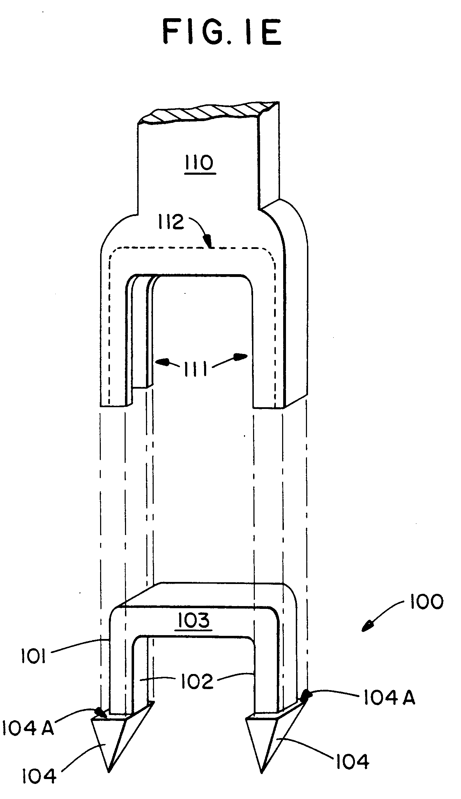

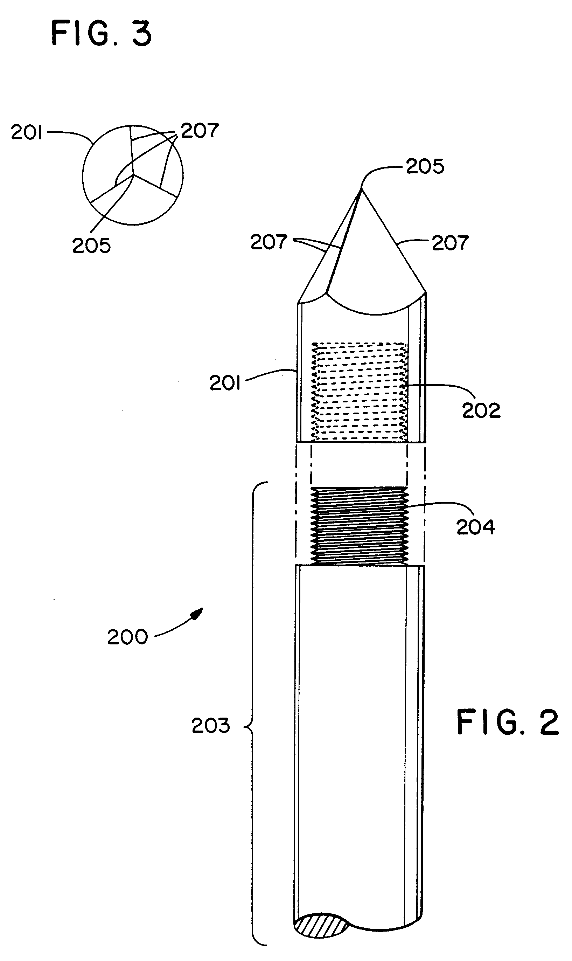

The basis of the present invention is the attachment of bone penetrating elements to bioabsorbable polymer implant devices, such as fasteners, thereby enabling the implant devices to be drilled or driven directly into bone or other hard tissue. Common types of bone fasteners include staples and pins, illustrated in the inventive embodiments by Figs. 1 and 2, respectively.

The fastening body portion of the bone fixation device of the present invention is fabricated from a biodegradable material such as one or more of the several types of bioabsorbable polymers commonly used in such applications. Examples include poly (p-dioxanone), polylactide, polyglycolides, polycaprolactone, poly (orthoesters) and the like, as well as copolymers of the same. Optionally, the biodegradable material used in the various embodiments of this invention, may contain reinforcing fibers, so as to produce a high strength composite. The reinforcing fibers can be, for example, polymeric, or ceramic materials, and either bioabsorbable or permanent.

The terms "biodegradable" and "bioabsorbable" are used interchangeably herein, and refer to materials which are chemically broken down and/or assimilated by human (or animal) body tissue.

The bone penetrating elements of the fastener of the present invention are preferably in the form of tips of metallic or ceramic material for initially contacting the bone and enabling the fastener to penetrate the bone when a suitable driving force is applied. The tips preferably have means for cutting bone or hard tissue such as a relatively sharp point or one or more sharp edges.

The tips should be of sufficient size and mass relative to the fastening body portion of the surgical fastener, to have the mechanical strength necessary to penetrate bone or hard tissue. However, because the material best suited for fabricating the tips is non-biodegradable, the optimum size of the tips is the minimum size necessary to perform its function of penetrating hard tissue and bone for those surgical applications in which the tips will remain embedded in the bone. As discussed below, not all surgical applications require the bone penetrating tips to remain embedded in bone.

Figs. 1A, 1B, 1C and 1D illustrate a staple type fastener 100 of the present invention, which comprises a fastening body portion 101 for fastening body tissue. The fastening body portion 101 is optimally an integral single piece construction having a crosspiece 103 and legs 102. Legs 102 extend substantially perpendicularly from the crosspiece 103.

The fastening body portion 101 as shown in Figs. 1A and 1C is U-shaped, with legs 102 extending from the ends of the crosspiece 103. Alternatively, the legs 102 may be spaced inward from the ends of crosspiece 103, the invention can have one or more legs, and although the legs 102 are illustrated as being of substantially equal length, legs of unequal length are also contemplated as being within the scope of this invention.

Unlike prior art fasteners, the fastening body portion 101 of the present surgical fastener 100 has one or more bone penetrating elements such as tips 104 attached to the distal ends of legs 102, for penetrating bone or hard tissue. The tips 104 must have a hardness sufficient for such penetration. Thus, tips 104 are preferably made of a metal, such as stainless steel, titanium and its alloys, cobalt-chromium-molybdenum alloys, or other implant grade metal alloys. Ceramics having appropriate hardness and toughness may also be used, such as zirconia, aluminum oxide, carbon/carbon composites, etc. The tips 104 each have a relatively sharp point 107 for easier bone penetration, but any appropriate shape which will perform the same penetrating function may be employed. Also the tips 104 may have backward pointing barbs (not shown) to prevent removal of the fastener.

Each tip 104 has a rearward projecting bolt 105 which engages and is received in a corresponding cavity 106 in the distal end of the respective leg 102. The tips 104 can be attached to the distal ends of the legs 102 by various alternative means. For example, the bolts 105 may be threaded, and cavity 106 may be tapped to form a screw fit. The joint is preferably secured by mounting the tips 104 additionally with a biocompatible adhesive. Or the tips 104 may be fused to the legs 102 by ultrasonic welding, or induction heating.

Referring to Figs. 1B and 1C, tips 104 have an impact surface 104A for receiving a driving force for implanting the fastener. The impact surface 104A is perpendicular to the direction of the driving force and extends outwardly beyond the surface of the legs 102 on three or fewer sides of each leg. The impact surface does not extend beyond the interior surfaces of the leg 102 into the space between the two legs. As can be seen from Figs. 1B, 1C and 1D, the edges of the tips 104 are flush with the inside surfaces of the legs 102, thereby preserving a tight fit with minimal latitude for loose movement or play in the fastener once implanted.

Tips 104 optionally have sharp points 107 and edges 108 for cutting through bone and hard tissue, so as to facilitate implantation of the fastener 100.

Fig. 1E illustrates the staple in conjunction with a pusher 110 for applying the staple to bone or hard tissue. Pusher 110 has legs 111 which are adapted to apply a driving force directly onto the projecting impact surface 104A of tips 104. Surface 112 engages the crosspiece 103 when impact is applied to keep the tips 104 from being driven apart from the ends of legs 102. As the tips penetrate bone this surface pushes the staple into the channels left by the tips. The driving force may be applied manually by the surgeon, or through the use of a powered instrument.

Figs. 2 and 3 illustrate another embodiment of the present invention. In this embodiment, surgical pin fastener 200 comprises a bioabsorbable fastening body portion in the form of a shaft 203, said body or shaft 203 having a bone penetrating element 201 attached thereto. Bone penetrating element 201 is optimally a hard trocar tip which is fixed to the shaft 203, and preferably has a relatively sharp point 205 and one or more relatively sharp edges 207, for cutting bone.

The trocar tip 201 may be constructed from any material having a hardness and strength sufficient for penetrating bone or hard tissue. Examples of appropriate metals and ceramic materials have been recited herein above.

The trocar tip 201 is preferably fixed to the end of shaft 203 by a screw mounting. Projecting screw portion 204 of shaft 203 is received into tapped aperture 202 in the tip 201. Screw portion 204 is preferably an integral part of shaft 203 made of the same bioabsorbable material. Optionally, the joint can be made permanent by adhesively bonding the threaded joint.

Alternative joining methods can be used both for the pin 200 and staple 100. For example, the tips 104 and 201 may be fashioned with a sleeve, the sleeve being crimped onto the fastener structure. Interlocking grooves in the tips 104 and 201 in conjunction with cooperating grooves in the legs 102 and shaft 203 respectively are also envisioned.

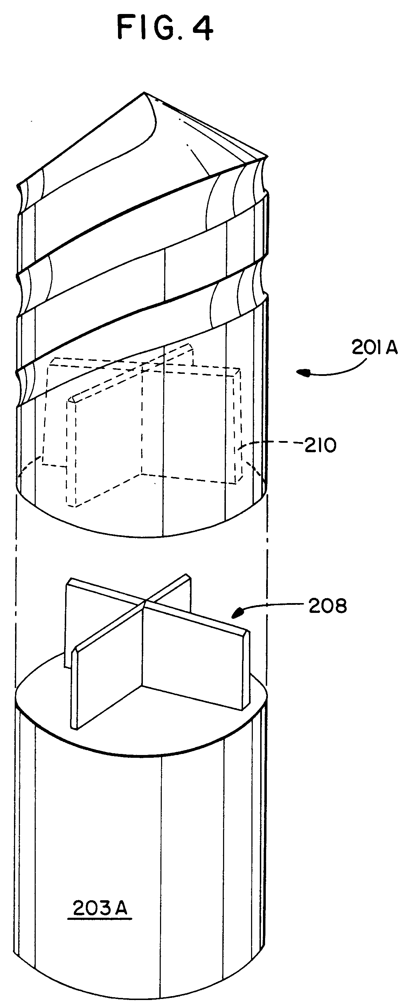

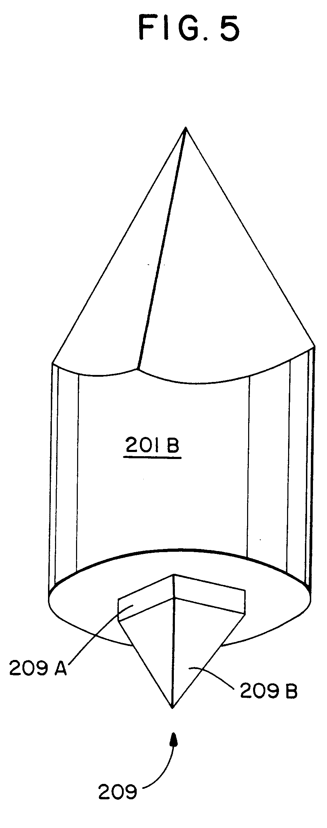

Fig. 4 illustrates alternative embodiments of the tip and shaft. Tip 201A can be fluted in order to function like a drill bit. The shaft 203A may have various types of connection or mounting fixtures, such as the substantially cross shaped male fixture 208 projecting perpendicularly from the end of the shaft, which is adapted to fit into the corresponding female receptacle 210 in the tip 201A. Or, as shown in Fig. 5, trocar tip 201B may have a male mounting fixture 209 adapted to fit into a female receptacle in the shaft. Male mounting fixture 209 optimally has a base portion 209A which projects perpendicularly from the bottom surface of tip 201B, and a pyramid portion 209B which culminates in a point. These tips and shafts may be mounted together adhesively or by welding or fusing the tips and shafts together. Those skilled in the art will envision other types of connection fixtures. It should be realized that the connecting fixtures 208 and 209 can also be used with the staple type bone fastener 100.

Typically, prior art metal bone fastening pins are drilled directly into the bone. Prior art bioabsorbable pins must have predrilled holes into which they are inserted. But in accordance with the present invention, self-inserting pin 200 can be implanted directly into bone by drilling, yet also has the advantage of being bioabsorbable. Alternatively, pin 200 may have a tip 201 with an impact surface, such as the impact surface 104A of the staple fastener 100.

The size of the surgical staple 100 and pin 200 may range from a few millimeters to several centimeters. However, the surgical bone fasteners of the present invention can be made of any size which is appropriate for its function of fastening bone or hard tissue.

Direct implantation of the bioabsorbable fasteners 100 and 200 is possible because the bone penetrating elements (tips 104 and 201) have a bone cutting means, such as relatively sharp points and edges which allow the bone penetrating elements (104 and 201) to pierce the bone when a driving force is applied to the fastener. The driving force can be an impulse or pushing force commonly used for driving staple type fasteners such as fastener 100, or a rotary or drilling force commonly used for pin type fasteners such as 200.

As can readily be seen, the method for using the present invention to fasten segments of bone or hard tissue is relatively simple. Being provided with the surgical fastener of the present invention, the surgeon implants the device into bone or hard tissue so that the bioabsorbable fastening body portion holds and maintains the segments of bone or hard tissue in close adjacency for sufficient period of time to promote healing. Both fasteners 100 and 200 will degrade over a period of time leaving only the metal tips 104 and 201, which, being small, are far less intrusive, and have little tendency to work loose from the bone and migrate.

In some surgical methods employing the fasteners of the present invention no hard nonabsorbable piece at all remains. For example, pin 200 can be driven completely across a fracture site so that the bone penetrating element emerges from the far side of the bone. In such a case, the bone penetrating tip will be cut off and removed, leaving no portion of the fastener which cannot be absorbed.

## Claims

1. A surgical fastener (100) comprising an elongate bioabsorbable fastening body portion (101) of uniform cross-section having at least one bone penetrating tip (104) of metallic material having a hardness sufficient for penetrating bone or hard tissue.

2. A surgical fastener (100) comprising an elongate bioabsorbable fastening body portion (101) of uniform cross-section having at least one bone penetrating tip (104) of ceramic material having a hardness sufficient for penetrating bone or hard tissue.

3. A fastener as claimed in claim 1 or 2, wherein said tip comprises means (108) for cutting bone or hard tissue.

4. A fastener as claimed in claim 3, wherein said means for cutting hard tissue is a relatively sharp point (107).

5. A fastener as claimed in claim 3, wherein said means for cutting hard tissue is at least one relatively sharp edge (108).

6. A fastener as claimed in any one of the preceding claims, wherein said fastening body portion comprises a leg (102) attached to a crosspiece (103).

7. A fastener as claimed in claim 6, wherein said leg extends substantially perpendicular from said crosspiece (103).

8. A fastener as claimed in any one of the preceding claims, wherein the bioabsorbable fastening body portion (101) is fabricated from a polymeric material.

9. A fastener as claimed in claim 8, wherein said polymeric material is selected from poly (p-dioxanone), polylactide, polyglycolide, polycaprolactone, and poly (orthoesters).

10. A fastener as claimed in claim 1 or any one of claims 3 to 9 as dependent on claim 1, wherein said metallic material is selected from stainless steel, titanium, titanium alloys and cobalt-chromium-molybdenum alloy.

11. A fastener as claimed in claim 2 or any one of claims 3 to 9 as dependent on claim 2, wherein said ceramic material is selected from zirconia, alumina, and carbon/carbon composites.

12. A fastener as claimed in any one of the preceding claims additionally having tip mounting means comprising a male mounting fixture (208) and a female receptacle (210) for receiving said male mounting fixture.

13. A fastener as claimed in claim 12, wherein said male mounting fixture (208) comprises a substantially cross-shaped member projecting axially from the end of said body portion.

14. A fastener as claimed in claim 12, wherein said male mounting fixture (209) comprises a base portion (209A) projecting perpendicular from the end surface of said bone penetrating tip (201B) and a substantially pyramidal portion (209B) projecting from said base portion.

15. A fastener as claimed in any one of the preceding claims, wherein the bioabsorbable fastening body portion contains a reinforcing material.

16. A fastener as claimed in claim 15, wherein the reinforcing material is at least one type of reinforcing fiber.

17. A fastener as claimed in claim 15 or 16, wherein the reinforcing material is chosen from polymeric resins and ceramics.

18. A fastener as claimed in any one of the preceding claims, wherein the bone penetrating tip comprises an impact surface (104A) for receiving a driving force.

## Patentansprüche

1. Chirurgische Befestigungsvorrichtung (100), umfassend ein langgestrecktes bioabsorbierbares Befestigungskörperteil (101) von einheitlichem Querschnitt mit mindestens einer Knochen durchdringenden Spitze (104) aus einem metallischen Werkstoff, der eine zum Durchdringen von Knochen oder Hartgewebe ausreichende Härte aufweist.

2. Chirurgische Befestigungsvorrichtung (100), umfassend ein langgestrecktes bioabsorbierbares Befestigungskörperteil (101) von einheitlichem Querschnitt mit mindestens einer Knochen durchdringenden Spitze (104) aus einem keramischen Werkstoff, der eine zum Durchdringen von Knochen oder Hartgewebe ausreichende Härte aufweist.

3. Befestigungsvorrichtung nach Anspruch 1 oder 2, dadurch gekennzeichnet, daß die besagte Spitze Mittel (108) zum Durchschneiden von Knochen oder Hartgewebe umfaßt.

4. Befestigungsvorrichtung nach Anspruch 3, dadurch gekennzeichnet, daß die besagten Mittel zum Durchschneiden von Hartgewebe eine relativ scharfe punktförmige Spitze (107) sind.

5. Befestigungsvorrichtung nach Anspruch 3, dadurch gekennzeichnet, daß die besagten Mittel zum Durchschneiden von Hartgewebe mindestens eine relativ scharfe Kante (108) sind.

6. Befestigungsvorrichtung nach einem beliebigen der vorangehenden Ansprüche, dadurch gekennzeichnet, daß das besagte Befestigungskörperteil ein an einem Querholm (103) befestigtes Bein (102) umfaßt.

7. Befestigungsvorrichtung nach Anspruch 6, dadurch gekennzeichnet, daß sich das besagte Bein im wesentlichen im rechten Winkel von dem besagten Querholm (103) weg erstreckt.

8. Befestigungsvorrichtung nach einem beliebigen der vorangehenden Ansprüche, dadurch gekennzeichnet, daß das bioabsorbierbare Befestigungskörperteil (101) aus einem Polymermaterial gefertigt ist.

9. Befestigungsvorrichtung nach Anspruch 8, dadurch gekennzeichnet, daß das besagte Polymermaterial aus Poly(p-dioxanon), Polylactid, Polyglycolid, Polycaprolacton und Poly(orthoestern) ausgewählt ist.

10. Befestigungsvorrichtung nach Anspruch 1 oder einem beliebigen der auf Anspruch 1 rückbezogenen Ansprüche 3 bis 9, dadurch gekennzeichnet, daß der besagte metallische Werkstoff aus Edelstahl, Titan, Titanlegierungen und Kobalt-Chrom-Molybdän-Legierungen ausgewählt ist.

11. Befestigungsvorrichtung nach Anspruch 2 oder einem beliebigen der auf Anspruch 2 rückbezogenen Ansprüche 3 bis 9, dadurch gekennzeichnet, daß der besagte keramische Werkstoff aus Zirkoniumoxid, Aluminiumoxid und Kohlenstoff/Kohlenstoffverbundwerkstoffen ausgewählt ist.

12. Befestigungsvorrichtung nach einem beliebigen der vorangehenden Ansprüche, dadurch gekennzeichnet, daß sie zusätzlich eine Spitzenmontageeinrichtung aufweist, die eine äußere Montage-Befestigungsvorrichtung (208) und eine innere Aufnahme (210) zum Aufnehmen der besagten äußeren Montage-Befestigungsvorrichtung umfaßt.

13. Befestigungsvorrichtung nach Anspruch 12, dadurch gekennzeichnet, daß die besagte äußere Montage-Befestigungsvorrichtung (208) ein axial aus dem Stirnende des besagten Körperteils vorstehendes, im wesentlichen kreuzförmiges Element umfaßt.

14. Befestigungsvorrichtung nach Anspruch 12, dadurch gekennzeichnet, daß die besagte äußere Montage-Befestigungsvorrichtung (209) ein senkrecht aus der Stirnfläche der besagten Knochen durchdringenden Spitze (201B) vorstehendes Unterteil und ein aus dem besagten Unterteil vorstehendes, im wesentlichen pyramidenförmiges Teil (209B) umfaßt.

15. Befestigungsvorrichtung nach einem beliebigen der vorangehenden Ansprüche, dadurch gekennzeichnet, daß das bioabsorbierbare Befestigungskörperteil ein Verstärkungsmaterial enthält.

16. Befestigungsvorrichtung nach Anspruch 15, dadurch gekennzeichnet, daß das Verstärkungsmaterial mindestens eine Art von Verstärkungsfaser ist.

17. Befestigungsvorrichtung nach Anspruch 15 oder 16, dadurch gekennzeichnet, daß das Verstärkungsmaterial aus Polymerharzen und keramischen Werkstoffen ausgewählt ist.

18. Befestigungsvorrichtung nach einem beliebigen der vorangehenden Ansprüche, dadurch gekennzeichnet, daß die Knochen durchdringende Spitze eine Stoßfläche (104A) zum Aufnehmen einer Treibkraft umfaßt.

## Revendications

1. Attache chirurgicale (100) comprenant une portion de corps d'attache biorésorbable allongée (101) d'une section transversale uniforme présentant au moins une pointe pénétrant dans l'os (104) en matériau métallique d'une dureté suffisante pour pénétrer dans l'os ou dans un tissu dur.

2. Attache chirurgicale (100) comprenant une portion de corps d'attache biorésorbable allongée (101) de section transversale uniforme présentant au moins une pointe pénétrant dans l'os (104) en matière céramique d'une dureté suffisante pour pénétrer dans l'os ou dans un tissu dur.

3. Attache selon la revendication 1 ou 2, dans laquelle ladite pointe comprend un moyen (108) pour couper l'os ou le tissu dur.

4. Attache selon la revendication 3, dans laquelle ledit moyen pour couper le tissu dur est une pointe relativement coupante (107).

5. Attache selon la revendication 3, dans laquelle ledit moyen pour couper le tissu dur est au moins un bord relativement coupant (108).

6. Attache selon l'une des revendications précédentes, dans laquelle ladite portion de corps d'attache comprend une branche (102) attachée à une traverse (103).

7. Attache selon la revendication 6, dans laquelle ladite branche s'étend de façon sensiblement perpendiculaire de ladite traverse (103).

8. Attache selon l'une des revendications précédentes, dans laquelle la portion de corps d'attache biorésorbable (101) est réalisée en matière polymère.

9. Attache selon la revendication 8, dans laquelle ladite matière polymère est choisie parmi les poly(p-dioxanone), polylactide, polyglycolide, polycaprolactone, et poly(orthoesters).

10. Attache selon la revendication 1 ou l'une des revendications 3 à 9 dépendant de la revendication 1, dans laquelle ledit matériau métallique est choisi parmi l'acier inoxydable, le titane, les alliages de titane et les alliages cobalt-chrome-molybdène.

11. Attache selon la revendication 2 ou l'une des revendications 3 à 9 dépendant de la revendication 2, dans laquelle ladite matière céramique est choisie parmi le zircone, l'oxyde d'aluminium et les composites carbone/carbone.

12. Attache selon l'une des revendications précédentes, présentant additionnellement un moyen de montage de pointe comprenant une fixation de montage mâle (208) et un logement femelle (210) pour recevoir ladite fixation de montage mâle.

13. Attache selon la revendication 12, dans laquelle ladite fixation de montage mâle (208) comprend un élément sensiblement en forme de croix faisant saillie axialement de l'extrémité de ladite portion de corps.

14. Attache selon la revendication 12, dans laquelle ladite fixation de montage mâle (209) comprend une portion de base (209A) faisant saillie perpendiculairement de la surface d'extrémité de ladite pointe de pénétration d'os (201B) et une portion sensiblement pyramidale (209B) faisant saillie depuis ladite portion de base.

15. Attache selon l'une des revendications précédentes, dans laquelle la portion de corps d'attache biorésorbable contient une matière de renforcement.

16. Attache selon la revendication 15, dans laquelle la matière de renforcement est constituée par au moins un type de fibre de renforcement.

17. Attache selon la revendication 15 ou 16, dans laquelle la matière de renforcement est choisie parmi des résines polymériques et des céramiques.

18. Attache selon l'une des revendications précédentes, dans laquelle la pointe de pénétration d'os comprend une surface d'impact (104A) pour recevoir une force d'entraînement.
